(19) Europäisches Patentamt
European Patent Office
Office européen des brevets

(11) Veröffentlichungsnummer: **0 446 621 B1**

# (12) EUROPÄISCHE PATENTSCHRIFT

(45) Veröffentlichungstag der Patentschrift: **07.12.94**

(51) Int. Cl.$^{5}$: **C08F 8/14**

(21) Anmeldenummer: **91101814.1**

(22) Anmeldetag: **09.02.91**

## (54) Polyacrylsäureester mit langkettigen alkoxylierten Kohlenwasserstoffoxy-Gruppen und deren Verwendung in der Kosmetik und Körperpflege.

(30) Priorität: **27.02.90 DE 4006093**

(43) Veröffentlichungstag der Anmeldung:
**18.09.91 Patentblatt 91/38**

(45) Bekanntmachung des Hinweises auf die Patenterteilung:
**07.12.94 Patentblatt 94/49**

(84) Benannte Vertragsstaaten:
**AT BE CH DE ES FR GB IT LI NL**

(56) Entgegenhaltungen:
**EP-A- 0 011 806**
**EP-A- 0 220 611**
**EP-A- 0 386 507**
**WO-A-86/04338**
**DE-B- 1 065 620**

(73) Patentinhaber: **Th. Goldschmidt AG**
**Goldschmidtstrasse 100**
**D-45127 Essen (DE)**

(72) Erfinder: **Esselborn, Eberhard**
**Pilotystrasse 21**
**W-4300 Essen 1 (DE)**
Erfinder: **Fock, Jürgen, Dr.**
**Mörsenbroicher Weg 114**
**W-4000 Düsseldorf 30 (DE)**
Erfinder: **Koerner, Götz, Dr.**
**Kantorie 126**
**W-4300 Essen (DE)**
Erfinder: **Schaefer, Dietmar, Dr.**
**Milchstrasse 40**
**W-4300 Essen 14 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 446 621 B1

Rank Xerox (UK) Business Services
(3.10/3.09/3.3.3)

## Beschreibung

Die Erfindung betrifft Polyacrylsäureester mit langkettigen alkoxylierten Kohlenwasserstoffoxy-Gruppen und deren Verwendung als Emulgatoren, Solubilisierungsmittel und als Verdickungsmittel für wäßrige, aniontensidhaltige Lösungen, insbesondere in der Kosmetik und Körperpflege.

Die Erfindung betrifft insbesondere Polyacrylsäureester mit einem verringerten Gehalt an niedrigmolekularen oder oligomeren Verbindungen sowie einer Verteilung der Polymeren, welche der Poisson-Verteilung angenähert ist, und insbesondere solche Polyacrylsäureester, die verbesserte physiologische Eigenschaften aufweisen.

Copolymerisate aus Polyoxyalkylen(meth)acrylsäureestern und Alkyl(meth)acrylsäureestern sind aus dem Stand der Technik bekannt.

So sind in der JP-OS 61/145254 organische und anorganische Pigmente beschrieben, deren Oberflächen mit einem Copolymerisat behandelt sind, welches aus 1 bis 99 Gew.-% Polyoxyalkylen(meth)acrylat und 1 bis 99 Gew.-% Alkylmethacrylat besteht, wobei der Alkylrest des Alkylmethacrylates 6 bis 22 Kohlenstoffatome aufweist. Diese Copolymerisate erhöhen die Dispergierbarkeit der mit ihnen behandelten Pigmente.

Aus der DE-OS 36 36 429 sind polymere Tenside auf der Basis von Copolymerisaten aus hydrophoben und hydrophilen Monomeren bekannt, welche

(a) als hydrophobe Monomere Verbindungen der Formel

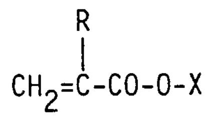

in der
R = H, $CH_3$
X = $C_4$- bis $C_{20}$-Alkyl,

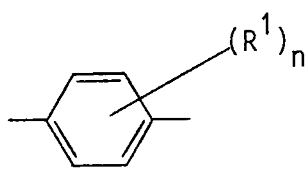

$R^1$ = H, $C_1$- bis $C_{20}$-Alkyl, Halogen,

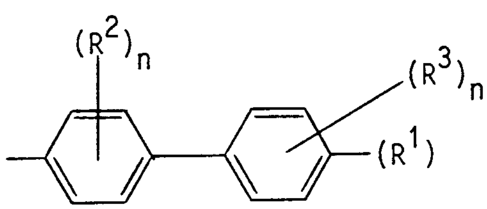

n = 1 bis 4
$R^2$, $R^3$ = H, $C_1$- bis $C_{20}$-Alkyl, F, Cl, Br
bedeutet, und

(b) als hydrophile Monomere Verbindungen der Formel

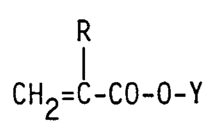

in der
R = H, $CH_3$,
Y =

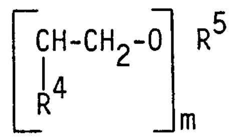

$R^4$ = H, $CH_3$, $C_2H_5$
$R^5$ = H, $C_1$- bis $C_4$-Alkyl und
m = 2 bis 50
bedeutet, einpolymerisiert enthalten.

Diese polymeren Tenside bilden in wäßrigen Systemen oberhalb der kritischen Micellkonzentration micellare Strukturen aus, die bei geeigneter Struktur und in bestimmten Konzentrationsbereichen als flüssigkristalline Phasen (Mesophasen) vorliegen. Die in der DE-OS 36 36 429 beschriebenen Copolymerisate sollen eine breite Variation von Mesophasenstrukturen und Stabilitätsparametern ermöglichen. Die Copolymerisate haben tensidische Eigenschaften. Die wasserlöslichen Verbindungen können in Waschmittelformulierungen zur Verstärkung der Waschkraft eingesetzt werden. Die Copolymerisate können ferner zur Erhöhung der Viskosität wäßriger Phasen dienen, wobei die viskositätserhöhende Wirkung weitgehend pH-unabhängig ist.

Die EP-OS 0 011 806 betrifft ein flüssiges Emulsionspolymerisat, welches pH-abhängig auf wäßrige Zubereitungen verdickend wirkt, enthaltend:

a) 15 - 60 Gew.-% wenigstens einer $\alpha,\beta$-ethylenisch ungesättigten monomeren Carbonsäure der Formel

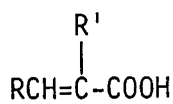

wobei

| | |
|---|---|
| R | entweder ein Wasserstoffrest und R' ein Wasserstoffrest, ein Alkylrest mit 1 bis 4 Kohlenstoffatomen oder |
| R | ein -COOX-Rest und R' ein Wasserstoffrest oder der Rest $-CH_2COOX$ oder |
| R | ein Methylrest und R' ein Wasserstoffrest ist, und |
| X | ein Wasserstoffrest oder ein Alkylrest mit 1 bis 4 Kohlenwasserstoffatomen ist, |

b) 15 - 80 Gew.-% wenigstens eines nichtionischen, copolymerisierbaren $\alpha,\beta$-ethylenisch ungesättigten Monomeren der Formel

$CH_2=CYZ$

wobei

| | |
|---|---|
| Y | entweder ein Wasserstoffrest und Z ein -COOR-, $-C_6H_4R'$-, -CN-, -Cl-, 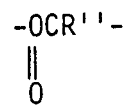 oder $-CH=CH_2$-Rest oder |
| Y | ein Methylrest und Z ein -COOR-, $-C_6H_4R'$-, -CN- oder $-CH=CH_2$-Rest oder |
| Y und Z | ein Chlorrest ist und |
| R | ein Alkylrest mit 1 bis 8 Kohlenstoffatomen oder ein Hydroxyalkylrest mit 2 bis 8 Kohlenstoffatomen ist, |
| R' | ein Wasserstoff-, Chlor-, Brom- oder Alkylrest mit 1 bis 4 Kohlenstoffatomen ist und |
| R'' | ein Alkylrest mit 1 bis 8 Kohlenstoffatomen ist, und |

c) 1 - 30 Gew.-% wenigstens eines nichtionischen oberflächenaktiven Vinylesters der Formel

$$R''O-(CH_2\overset{\overset{\displaystyle R'}{|}}{C}HO)_m(C_2H_4O)_n-\overset{\overset{\displaystyle O}{\|}}{C}-\overset{\overset{\displaystyle R}{|}}{C}=CH_2$$

wobei

- R ein Wasserstoff- oder Methylrest ist, jeder R'-Rest ein Alkylrest mit 1 oder 2 Kohlenstoffatomen ist,
- R'' ein Alkylrest mit 8 bis 20 Kohlenstoffatomen oder ein Alkylphenylrest mit 8 bis 16 Kohlenstoffatomen ist,
- n im Durchschnitt eine Zahl von 6 bis 100 und m im Durchschnitt eine Zahl von 0 bis 50 ist, mit der Maßgabe, daß $n \geqq m$ und $(n + m)$ gleich 6 bis 100 ist;

wobei das Polymere in Form einer wäßrigen kolloidalen Dispersion bei einem pH-Wert < etwa 5,0 stabil ist, aber bei Einstellung eines pH-Wertes von 5,5 bis 10,5 und höher ein wirkungsvoller Verdicker für wäßrige Systeme wird.

Copolymerisate ähnlicher Zusammensetzung sind in der US-PS 4 469 611 und der US-PS 4 552 685 beschrieben. Die gelchromatographische Untersuchung dieser Copolymerisate des Standes der Technik zeigt eine relativ breite Molekulargewichtsverteilung, welche für eine verminderte tensidische Wirksamkeit verantwortlich sein kann. Wie sich weiterhin durch NMR-spektroskopische und gaschromatographische Analyse nachweisen läßt, enthalten die Copolymerisate erhebliche Anteile an niedrigmolekularen Verbindungen. Diese niedrigmolekularen Anteile bestehen aus erheblichen Gehalten an Restmonomeren sowie niedrigmolekularen Verbindungen. Es wird angenommen, daß der Grund hierfür in den stark voneinander abweichenden Polymerisationsparametern der verschiedenen Monomeren zu suchen ist.

Diese niedrigmolekularen Anteile sind jedoch in mehrfacher Weise unerwünscht. Diese niedrigmolekularen Anteile beeinträchtigen und vermindern die grenzflächenaktiven Eigenschaften der bekannten Copolymerisate, da sie selbst zu diesen Eigenschaften nichts oder nur wenig beitragen. Die niedrigmolekularen Anteile sind aber auch aus physiologischen Gründen unerwünscht, da sie zu gesundheitlichen Beeinträchtigungen, wie Hautreizungen, Sensibilisierungen, etc. führen können.

Eine Abtrennung dieser störenden Anteile aus den Copolymerisaten des Standes der Technik ist in wirtschaftlicher Weise nicht möglich.

Der Erfindung liegt die Aufgabe zugrunde, Polyacrylsäureester mit langkettigen Kohlenwasserstoff- und Polyoxyalkylengruppen bereitzustellen, welche einerseits als Emulgatoren, Solubilisierungsmittel und zum Verdicken wäßriger, aniontensidhaltiger Zubereitungen verwendet werden können und deren physiologische Eigenschaften andererseits wesentlich verbessert sind. Dies soll insbesondere dadurch erreicht werden, daß die vorgenannten Polyacrylsäureester eine möglichst enge Molekulargewichtsverteilung besitzen und möglichst frei von monomeren und/oder niedrigmolekularen Anteilen sind.

Diese Eigenschaften wurden nun erfindungsgemäß bei solchen Polyacrylsäureestern gefunden, welche durch Umesterung von durch radikalische Polymerisation erhaltenen Polyacrylsäurealkylestern, deren Alkylgruppen 1 bis 8 Kohlenstoffatome aufweisen, wobei bis zu 50 % der Acrylsäureester durch die entsprechenden Methacrylsäureester ersetzt sein können, mit Polyoxyalkylenmonoolen der allgemeinen durchschnittlichen Formel

$R^1O\text{-}(C_nH_{2n}O\text{-})_xH$ I

erhältlich sind,

wobei

- $R^1$ ein Alkylrest mit 8 bis 30 Kohlenstoffatomen, ein Alkenylrest mit 8 bis 22 Kohlenstoffatomen oder ein Mono- oder Dialkylphenylrest mit 6 bis 16 Kohlestoffatomen je Alkylrest ist, wobei bis zu 40 % der Reste $R^1$ durch Alkylreste mit 1 bis 4 Kohlenstoffatomen ersetzt sein können,
- n einen Wert von 2, 3 oder 4 hat und im durchschnittlichen Molekül einen mittleren Wert von 2,0 bis 2,5 aufweist, und
- x einen Wert von 10 bis 200 hat,

in solchen Mengen, daß 5 bis 70 % der Estergruppen umgeestert werden und in Gegenwart eines an sich

bekannten Umesterungskatalysators, bei Temperaturen von 70 bis 170 °C und gegebenenfalls in Gegenwart eines Lösungsmittels.

Ausgangsverbindungen sind somit Polyacrylsäurealkylester, die durch radikalische Polymerisation hergestellt sind. Die Alkylgruppen der Polyacrylsäurealkylester weisen 1 bis 8 Kohlenstoffatome auf und sind vorzugsweise geradkettig. Besonders bevorzugt sind Alkylgruppen mit 1 bis 4 Kohlenstoffatomen, wie die Methyl-, Ethyl-, Propyl- und Butylgruppe. Insbesondere sind die Methyl- und Ethylgruppe bevorzugt, da das im Verlauf der Umesterung freiwerdende Methanol bzw. Ethanol am einfachsten, zum Beispiel durch Destillation, aus dem Reaktionsgemisch entfernt werden kann. Entscheidend ist dabei, daß die durch radikalische Polymerisation erhaltenen Polyacrylsäureester im wesentlichen frei von niedrigmolekularen Anteilen sind und eine Mclekulargewichtsverteilung aufweisen, die der Poisson-Verteilung zumindest annähernd entspricht. Diese Verteilung bleibt bei der Umesterung erhalten, so daß die erfindungsgemäßen Produkte, im Gegensatz zu den durch Copolymerisation eines Monomerengemisches erhaltenen Produkten, ebenfalls im wesentlichen frei von niedrigmolekularen Produkten oder oligomeren Anteilen sind.

Die erfindungsgemäßen Verbindungen sind dadurch erhältlich, daß man diese Polyacrylsäurealkylester mit Polyoxyalkylenmonoolen der allgemeinen durchschnittlichen Formel

$$R^1O\text{-}(C_nH_{2n}O\text{-})_xH \qquad I$$

umestert. Dabei haben die Reste und Indices folgende Bedeutung bzw. Werte:

$R^1$ kann gleich oder verschieden sein und einen Alkylrest, einen Alkenylrest oder einen Mono- oder Dialkylphenylrest bedeuten.

Der Alkylrest hat 8 bis 30 Kohlenstoffatome. Beispiele solcher Alkylreste sind der Octyl-, Decyl-, Undecyl-, Dodecyl-, Stearyl- oder Behenylrest. Bevorzugt sind Alkylreste mit 14 bis 22 Kohlenstoffatomen und insbesondere solche, die sich von den Fettalkoholen herleiten, die aus natürlichen Fettsäuren durch Reduktion erhalten worden sind.

Der Alkenylrest hat 8 bis 22 Kohlenstoffatome, vorzugweise 8 bis 18 Kohlenstoffatome. Besonders bevorzugt ist der Alkenylrest eines ungesättigten Fettalkohols, wie etwa der Oleyl- oder Linoleylrest.

Der Mono- oder Dialkylphenylrest weist in der oder den Alkylgruppen jeweils 6 bis 16 Kohlenstoffatome auf. Bevorzugte Alkylphenylreste sind der Mono- oder Dioctyl-, -nonyl-, -decyl- oder -dodecylphenylrest.

Es wird jedoch zugelassen, daß bis zu 40 % der Reste $R^1$ durch Alkylreste mit 1 bis 4 Kohlenstoffatomen ersetzt sein können.

In der allgemeinen Formel I gibt n die Anzahl der Kohlenstoffatome der Oxyalkylengruppen an. n hat dabei je Oxyalkyleneinheit einen Wert von 2, 3 oder 4. Es handelt sich also um Oxyethylen-, Oxypropylen- und Oxybutyleneinheiten. Im durchschnittlichen Molekül hat n einen mittleren Wert von 2,0 bis 2,5, vorzugsweise von 2,0 bis 2,3. Hierdurch ist eine Mindesthydrophilie der erfindungsgemäßen Verbindungen gewährleistet.

Der Index x entspricht der Anzahl der Oxyalkyleneinheiten und hat einen Wert von 10 bis 200, vorzugsweise von 40 bis 60. Auch hierbei handelt es sich um durchschnittliche Werte.

Die Polyoxyalkylenmonoole der Formel I werden mit den Polyacrylsäurealkylestern in solchen Mengenverhältnissen umgesetzt, daß 5 bis 70 %, vorzugsweise 20 bis 70 %, insbesondere 30 bis 70 % der Estergruppen umgeestert werden. Der Grad der Umesterung läßt sich aus der Menge des vom Polyacrylsäurealkylester stammenden Alkanols verfolgen.

Die Umesterung erfolgt in Gegenwart eines Umesterungskatalysators. Derartige Katalysatoren sind dem Fachmann bekannt. Beispiele solcher Katalysatoren sind i-Propyl- oder n-Butyltitanat, Kalium- oder Natriummethylat, p-Toluolsulfonsäure, Methansulfonsäure oder Trifluoressigsäure.

Es ist meist vorteilhaft, die Umsetzung in einem Lösungsmittel durchzuführen. Bevorzugte Lösungsmittel sind Toluol oder Xylol.

Die Umesterungstemperatur beträgt etwa 70 bis 170 °C, insbesondere etwa 90 bis 150 °C.

Durch die Wahl der Reste $R^1$ sowie der Polymerindices x und n werden Wasserlöslichkeit, Hydrophilie, Solubilisierungsvermögen und Verträglichkeit mit anionischen Tensiden beeinflußt.

Ganz allgemein läßt sich feststellen, daß mit zunehmendem Gehalt an Ethylenoxid und abnehmender Kohlenstoffzahl des Alkyl- oder Alkenylrestes die Wasserlöslichkeit zunimmt. In diesem Zusammenhang ist es auch günstig, bis zu 40 % der Reste $R^1$ durch Alkylreste mit 1 bis 4 Kohlenstoffatomen zu ersetzen.

Bis zu 50 Mol-% der Acrylsäurealkylester können durch die entsprechenden Methacrylsäurealkylester ersetzt sein.

Es ist dem Fachmann klar, daß die als Ausgangsverbindungen für die Umesterungsreaktion dienenden Polyacrylsäurealkylester auch andere Comonomere einpolymerisiert enthalten können, wie Styrol, Acrylamid, Acrylnitril oder Methacrylsäurealkylester.

Zur Verwendung der erfindungsgemäßen Verbindungen als Solubilisatoren und Emulgatoren bzw. Coemulgatoren sind insbesondere solche Polymere besonders geeignet, in denen $R^1$ ein Alkyl- oder Alkenylrest mit mindestens 14 Kohlenstoffatomen oder ein Mono- bzw. Dialkylphenylrest, dessen Alkylrest 6 bis 16 Kohlenstoffatome aufweist, ist.

Bei der Verwendung der erfindungsgemäßen Verbindungen als Emulgatoren ist zu beachten, daß die Hydrophilie des Emulgators der jeweils zu emulgierenden Ölphase angepaßt werden muß (HLB-Prinzip). Die Einstellung der benötigten Hydrophilie (HLB-Wert) ist durch geeignete Auswahl der Polymerindices n und x möglich. Im allgemeinen werden solche Polymeren bevorzugt, in denen n einen Wert zwischen 2 und 2,5 annimmt. Der Index x nimmt in diesem Falle üblicherweise einen Wert von 10 bis 50 an.

Hinsichtlich der verdickenden Wirkung der erfindungsgemäßen Verbindungen in Gegenwart von Aniontensiden (Shampooformulierungen) werden solche Produkte bevorzugt, in denen $R^1$ ein Alkyl- oder Alkenylrest mit 16 bis 22 Kohlenstoffatomen ist. Als vorteilhaft hat sich hier die Verwendung von Polyethylenoxidsegmenten mit 30 bis 60 Monomereinheiten erwiesen. Die Verwendung von Propylenoxid als Comonomeres neben Ethylenoxid in den Polyethersegmenten senkt die Verträglichkeit mit Aniontensiden. Durch Einbringen relativ geringer Mengen Propylenoxid läßt sich jedoch eine Kristallisation der erfindungsgemäßen Verbindungen verhindern und damit eine verbesserte Handhabbarkeit erreichen. Mit der gleichen Zielsetzung lassen sich auch kleine Mengen Butylenoxid verwenden. Die Verträglichkeit mit Aniontensiden läßt sich darüber hinaus verbessern, wenn zusätzlich zu den längerkettigen ethoxylierten Fettalkoholen auch Ethoxylate von niedrigen aliphatischen Alkoholen, wie z.B. Methanol, bei der Umesterung eingesetzt werden.

In den folgenden Beispielen wird die Herstellung der erfindungsgemäßen Verbindungen und deren verdickende Wirkung gezeigt.

## Beispiel 1

Herstellung von Polymethylacrylat durch radikalische Polymerisation (nicht erfindungsgemäß)

Eine Lösung von 0,6 g Azodiisobuttersäurenitril und 20,2 g Dodecylmercaptan in 50 g Toluol und 280 g (ca. 3,25 Mol) Methylacrylat wird innerhalb von 2 h in einen mit 53 g Toluol gefüllten Reaktor gegeben; das vorgelegte Lösungsmittel hat dabei eine Temperatur von 100 °C und befindet sich unter einer Stickstoffatmosphäre. Danach werden nochmal 0,9 g Azodiisobuttersäurenitril, gelöst in 20 g Methylethylketon, innerhalb von 0,5 h nachgegeben. Schließlich wird das Reaktionsgemisch noch für 1 h bei der gleichbleibenden Temperatur von 100 °C weiter erwärmt. Nach Beendigung der Reaktion wird das Lösungsmittel abdestilliert. Es verbleibt eine farblose, viskose Flüssigkeit mit einem Brechungsindex von 1,4802. Aus der gelchromatographischen Untersuchung ergibt sich für das erhaltene Polymerisat ein numerisches Molekulargewicht $\overline{M}_n$ von 1950 und für das Gewichtsmittel des Molekulargewichtes $\overline{M}_w$ 3330; der Uneinheitlichkeitskoeffizient beträgt demnach 1,71. Der Restmonomerengehalt beträgt < 0,1 %.

## Beispiele 2 bis 4

Herstellung von Polymethylacrylaten unterschiedlichen Molekulargewichtes durch radikalische Polymerisation (nicht erfindungsgemäß)

Es wird verfahren wie in Beispiel 1 mit der Ausnahme, daß der Gehalt an Dodecylmercaptan gesenkt wird. In Tabelle 1 wird die Abhängigkeit der Zahlen- und Gewichtsmittel des Molekulargewichtes vom Gehalt an Dodecylmercaptan gezeigt. Der gefundene Restmonomerengehalt beträgt in beiden Fällen < 0,1 %.

Tabelle 1

| Polymethylacrylat aus Beispiel | Dodecylmercaptan [Gew.-%] | Molekulargewicht $\overline{M}_n$ | Molekulargewicht $\overline{M}_w$ | Uneinheitlichkeitskoeffizient |
|---|---|---|---|---|
| 2 | 13,5 | 836 | 1 302 | 1,56 |
| 3 | 2,95 | 4 453 | 11 346 | 2,55 |
| 4 | 0,43 | 16 750 | 68 500 | 4,09 |

Beispiel 5

Herstellung von Poly-n-butylacrylat durch radikalische Polymerisation (nicht erfindungsgemäß)

Es wird verfahren wie in Beispiel 1 mit dem Unterschied, daß anstelle von Methylacrylat n-Butylacrylat eingesetzt wird.

Aus der gaschromatographischen Untersuchung ergibt sich für das erhaltene Polymerisat ein numerisches Molekulargewicht $\overline{M}_n$ von 1900 und für das Gewichtsmittel des Molekulargewichtes $\overline{M}_w$ 3300; der Uneinheitlichkeitskoeffizient beträgt demnach 1,73. Der Restmonomerengehalt wird zu < 0,1 % ermittelt.

Beispiel 6

Herstellung von Poly-2-ethylhexylacrylat durch radikalische Polymerisation (nicht erfindungsgemäß)

Es wird verfahren wie in Beispiel 1 mit dem Unterschied, daß anstelle von Methylacrylat 2-Ethylhexylacrylat eingesetzt wird.

Aus der gaschromatographischen Untersuchung ergibt sich für daserhaltene Polymerisat ein numerisches Molekulargewicht $\overline{M}_n$ von 1800 und für das Gewichtsmittel des Molekulargewichtes $\overline{M}_w$ 3030; der Uneinheitlichkeitskoeffizient beträgt demnach 1,68. Der Restmonomerengehalt wird zu ca. 0,1 % ermittelt.

Beispiel 7

Herstellung eines Methylacrylat-Methylmethacrylat-Copolymerisates durch radikalische Polymerisation (nicht erfindungsgemäß)

Es wird verfahren wie in Beispiel 1 mit der Ausnahme, daß anstelle einer Menge von 280 g (ca. 3,25 Mol) Methylacrylat 140 g (ca. 1,61 Mol) Methylacrylat und 140 g (ca. 1,4 Mol) Methylmethacrylat eingesetzt werden. Aus der gelchromatographischen Untersuchung ergibt sich für das erhaltene Polymerisat ein numerisches Molekulargewicht $\overline{M}_n$ von 2280 und für das Gewichtsmittel des Molekulargewichtes $\overline{M}_w$ 4390; der Uneinheitlichkeitskoeffizient beträgt demnach 1,93. Der Restmonomerengehalt wird zu etwa 0,15 % ermittelt.

Beispiel 8

Herstellung eines Oleyloxypolyethylenoxidmonools (nicht erfindungsgemäß)

268 g (ca. 1 Mol) Oleylalkohol und 7,0 g (ca. 0,1 Mol) Kaliummethylat werden in einen Reaktor gegeben. Nach sorgfältiger Spülung mit Reinstickstoff wird auf 110 °C geheizt und 2420 g (ca. 55 Mol) Ethylenoxid so schnell zugegeben, daß die Reaktorinnentemperatur 120 °C und der Druck 6 bar nicht überschreiten. Nach vollständiger Einleitung des Ethylenoxids wird die Temperatur so lange auf 115 °C gehalten, bis daß gleichbleibender Druck das Ende der Nachreaktion anzeigt. Anschließend werden 95,7 g (ca. 1,65 Mol) Propylenoxid zugegeben und nochmals bei 115 °C bis zur Druckkonstanz gehalten. Schließlich werden bei 80 bis 90 °C die nicht umgesetzten Monomeren unter Vakuum entfernt.

Das erhaltene Produkt wird mit Hilfe von verdünnter Phosphorsäure neutralisiert und das Wasser durch Destillation, das entstandene Kaliumphosphat durch Filtration zusammen mit einem Filterhilfsmittel entfernt. Das aus der Bestimmung der Hydroxylzahl bei einer angenommenen Funktionalität von 1 ermittelte Molekulargewicht beträgt 2310.

Beispiele 9 bis 25

Herstellung verschiedener Alkyloxy- bzw. Alkylaryloxypolyalkylenoxidmonoole (nicht erfindungsgemäß)

Die Herstellung und Aufarbeitung verschiedener Alkoxylate von Alkanolen und von Alkylphenolen erfolgt grundsätzlich in der in Beispiel 8 beschriebenen Weise durch Anlagerung der Alkylenoxide in Gegenwart alkalischer Katalysatoren und anschließende Neutralisation mit verdünnter Phosphorsäure. Beispiele der verwendeten Startalkohole, der Alkylenoxide nach Art und Molzahl sowie die durch Bestimmung der Hydroxylzahl ermittelten Molekulargewichte befinden sich in der Tabelle 2.

Tabelle 2

| Polyalkylenoxid Beispiel Nr. | Starter | EO [Mol] | PO [Mol] | BO [Mol] | MG [OHZ] |
|---|---|---|---|---|---|
| 8 | OLA | 55 | 1,65* | - | 2 310 |
| 9 | OLA | 10 | - | - | 646 |
| 10 | OLA | 25 | 19,0 | - | 2 040 |
| 11 | OLA | 40 | 8,5 | - | 2 280 |
| 12 | OLA | 44 | - | 3,7 | 2 149 |
| 13 | TFA | 2 | - | - | 352 |
| 14 | TFA | 20 | - | - | 1 152 |
| 15 | TFA | 50 | 1,5* | - | 2 040 |
| 16 | BHA | 50 | 1,5* | - | 2 290 |
| 17 | C21 | 50 | 1,5* | - | 2 377 |
| 18 | CTA | 50 | 1,5* | - | 2 440 |
| 19 | DDO | 50 | 1,5* | - | 2 226 |
| 20 | OCA | 20 | 1,5* | - | 967 |
| 21 | MEA | 21 | 1,5* | - | 949 |
| 22 | MEA | 43 | 1,5* | - | 1 970 |
| 23 | NP | 10 | - | - | 660 |
| 24 | NP | 50 | - | - | 2 550 |
| 25 | DNP | 49 | - | - | 1 982 |

* Propylenoxid am hydroxyfunktionellen Kettenende

Legende:

MEA = Methanol
OCA = Octanol-1
DDO = Dodecanol-1
CTA = Cetylalkohol
OLA = Oleylalkohol
TFA = Talgfettalkohol
C21 = Exxal 21®
BHA = Behenylalkohol
NP = Nonylphenol
DNP = Dinonylphenol

Beispiel 26

Herstellung eines Umesterungsproduktes aus Polymethylacrylat und alkoxyliertem Oleylalkohol

92,3 g des Polymethylacrylates aus Beispiel 1, gelöst in 554 g Toluol, werden zusammen mit 462 g (ca. 0,2 Mol) eines Oleyloxypolyethers gemäß Beispiel 8 unter Stickstoff erhitzt. Zunächst werden möglicherweise vorhandene Spuren von Wasser durch azeotrope Destillation entfernt. Danach erfolgt die Zugabe von 2 g Isopropyltitanat. Das bei der Umesterung entstehende Methanol wird durch Fraktionierung von Toluol getrennt. Nach 2 und 4 h Reaktionsdauer werden jeweils 2 g Isopropyltitanat nachdosiert. Die Reaktion ist nach etwa 6 h beendet; das Ende wird durch eine Kopftemperatur von etwa 110 °C angezeigt.

Der durch gelpermeationschromatographische Untersuchung ermittelte Anteil an nichtumgesetztem Oleyloxypolyether beträgt 125 g, entsprechend einem Umsatz von 72,9 %. Der Methanolgehalt im Destillat beträgt 4,36 g, entsprechend einem Umsatz von 68,1 % der Theorie. Hieraus ergibt sich, daß ca. 70,5 % der Estergruppen des Polymethacrylates umgesetzt worden sind. Die Farbzahl des Produktes nach Gardner beträgt nach Entfernung des Umesterungskatalysators durch Hydrolyse und Filtration 2 bis 3.

Beispiele 27 bis 49

Umesterungen von verschiedenen Polyacrylaten mit verschiedenen Fettalkoholalkoxylaten

Es wird verfahren wie in Beispiel 26 mit der Ausnahme, daß Polyacrylate unterschiedlichen Molekulargewichtes und unterschiedlicher Kohlenstoffzahl sowie in einem Fall ein Methylacrylat-Methylmethacrylat-Copolymerisat als estergruppenhaltige Komponente (s. Beispiele 1 bis 7) zum Einsatz kommen. Weiterhin werden unterschiedliche Fettalkoholalkoxylate eingesetzt, die sich in bezug auf die Kohlenstoffzahl des Alkyl-, Alkylacryl- bzw. Alkenylrestes auf das Molekulargewicht des Polyethersegmentes und dessen Zusammensetzung unterscheiden (s. Beispiele 8 bis 25). Der Substitutionsgrad der Theorie bezeichnet den Quotienten aus der Zahl der substituierten Estergruppen und der Zahl der ursprünglich vorhandenen Estergruppen. Der Substitutionsgrad der Praxis ergibt sich aus der gelpermeationschromatographischen Untersuchung bzw. aus der Menge an abgeschiedenem niedermolekularen Alkohol. Art und Menge der Komponenten sowie theoretischer und praktischer Substitutionsgrad sind in Tabelle 3 angegeben. Bei Verwendung von Poly-2-ethylhexylacrylat wird die Umesterung ohne Einsatz eines Lösungsmittels bei vermindertem Druck durchgeführt.

Beispiele 50 bis 53

Herstellung von Umesterungsprodukten unter Verwendung zweier unterschiedlicher Polyalkylenoxidmonoole (Coumesterung)

Es wird verfahren wie in Beispiel 26 mit der Ausnahme, daß zwei unterschiedliche Polyethermonoole gleichzeitig zum Einsatz kommen (Coumesterung). Art und Menge des Polyalkylenoxidmonools sowie der theoretische und praktische Substitutionsgrad sind in Tabelle 3 angegeben.

Tabelle 3

| Beispiel Nr. | Polyacrylat Beispiel Nr. | Polyacrylat Menge [g] | Polyalkylenoxid Beispiel Nr. | Polyalkylenoxid Menge [g] | Substitutionsgrad theor. [Mol-%] | Substitutionsgrad prakt. [Mol-%] |
|---|---|---|---|---|---|---|
| 27 | 1 | 92,3 | 9 | 129,2 | 20 | 19,0 |
| 28 | 1 | 92,3 | 10 | 408,0 | 20 | 6,4 |
| 29 | 1 | 92,3 | 11 | 456,0 | 20 | 13,2 |
| 30 | 1 | 92,3 | 12 | 429,8 | 20 | 12,6 |
| 31 | 1 | 92,3 | 8 | 693,0 | 30 | 21,1 |
| 32 | 1 | 92,3 | 8 | 924,0 | 40 | 26,5 |
| 33 | 1 | 92,3 | 8 | 1155,0 | 50 | 34,0 |
| 34 | 3 | 88,7 | 8 | 462,0 | 20 | 13,4 |
| 35 | 2 | 99,5 | 8 | 462,0 | 20 | 16,8 |
| 36 | 4 | 86,7 | 8 | 462,0 | 20 | 13,3 |
| 37 | 1 | 92,3 | 13 | 70,4 | 20 | 19,0 |
| 38 | 1 | 92,3 | 14 | 230,4 | 20 | 18,0 |
| 39 | 1 | 92,3 | 15 | 408,0 | 20 | 15,7 |
| 40 | 1 | 92,3 | 20 | 193,4 | 20 | 17,5 |
| 41 | 1 | 92,3 | 19 | 445,2 | 20 | 15,5 |
| 42 | 1 | 92,3 | 18 | 488,0 | 20 | 14,2 |
| 43 | 1 | 92,3 | 17 | 475,4 | 20 | 14,6 |
| 44 | 1 | 92,3 | 16 | 458,0 | 20 | 14,5 |
| 45 | 1 | 92,3 | 23 | 132,0 | 20 | 17,6 |
| 46 | 1 | 92,3 | 24 | 510,0 | 20 | 13,9 |
| 47 | 1 | 92,3 | 25 | 396,4 | 20 | 15,7 |
| 48 | 7 | 184,6 | 8 | 346,5 | 15 | 11,2 |
| 49 | 5 | 137,5 | 8 | 462,0 | 20 | 13,8 |

Tabelle 3 - Fortsetzung

| Beispiel Nr. | Polyacrylat Beispiel Nr. | Polyacrylat Menge [g] | Polyalkylenoxid Beispiel Nr. | Polyalkylenoxid Menge [g] | Substitutionsgrad theor. [Mol-%] | Substitutionsgrad prakt. [Mol-%] |
|---|---|---|---|---|---|---|
| 50 | 1 | 92,3 | 8/21 | 367/ 29 | 20 | 15,1 |
| 51 | 1 | 92,3 | 8/21 | 302/ 57 | 20 | 15,0 |
| 52* | 1 | 92,3 | 8/22 | 367/ 59 | 20 | 14,8 |
| 53 | 1 | 92,3 | 8/22 | 302/118 | 20 | 15,3 |
| 54 | 6 | 197,3 | 24 | 306 | 12 | 6,5 |
| 55* | 1 | 92,3 | 22 | 394 | 20 | 15,2 |

* = Vergleichsbeispiel

## Anwendungsbeispiele

Zum Nachweis der verdickenden Wirkung der erfindungsgemäßen Polyacrylsäureester mit langkettigen alkoxylierten Kohlenwasserstoffoxy-Gruppen werden diese als 40 %ige Lösungen in 1,2-Propandiol/Wasser ( = 2/1) in nachstehender Shampooformulierung eingesetzt:

| Phase A: | |
|---|---|
| erfindungsgemäße Verdickungsmittel (40 %):<br>Natriumlaurylethersulfat (28 %):<br>Parfümöl (Levona, Düllberg) : | x %<br>43 %<br>0,5 % |

| Phase B: | |
|---|---|
| Tego Betain LT [1]:<br>Wasser: | 10 %<br>ad 100 % |

[1] Tego Betain L7 = Cocamidopropyl-Betain (1-Alkoylamino-3-dimethylammonium-propan-3-carboxymethyl-betain)

Zur Verarbeitung werden zunächst bei Raumtemperatur die Phasen A und B getrennt hergestellt und anschließend Phase B unter kräftigem Rühren zur Phase A gegeben.

Die erhaltenen Viskositäten sind nachstehend zusammengefaßt:

| erfindungsgemäßes Verdickungsmittel von Beispiel | Einsatzkonzentration [%] | Viskosität (25 °C, Brookfield LVF) [mPa.s] |
|---|---|---|
| 26 | 1,0<br>2,0<br>3,0 | 4 800<br>9 600<br>19 000 |
| 31 | 1,0<br>2,0 | 1 880<br>9 500 |
| 33 | 1,0<br>2,0 | 2 900<br>14 000 |
| 34 | 2,0<br>3,0 | 8 900<br>21 000 |
| 39 | 1,0<br>2,0<br>3,0 | 2 000<br>9 500<br>19 000 |
| 42 | 1,0<br>2,0<br>3,0 | 1 700<br>8 500<br>17 500 |
| 44 | 1,0<br>2,0<br>3,0 | 2 000<br>9 500<br>20 500 |
| 55 (Vergleich) | 3,0 | 8 000 |

**Patentansprüche**

**1.** Polyacrylsäureester mit langkettigen alkoxylierten Kohlenwasserstoffoxy-Gruppen, erhältlich durch Umesterung von durch radikalische Polymerisation erhaltenen Polyacrylsäurealkylestern, deren Alkylgruppen 1 bis 8 Kohlenstoffatome aufweisen, wobei bis zu 50 % der Acrylsäureester durch die entsprechenden Methacrylsäureester ersetzt sein können, mit Polyoxyalkylenmonoolen der allgemeinen durchschnittlichen Formel

$R^1O\text{-}(C_nH_{2n}O\text{-})_xH$

wobei

$R^1$ im Polymeren gleich oder verschieden sein kann und ein Alkylrest mit 8 bis 30 Kohlenstoffatomen,
ein Alkenylrest mit 8 bis 22 Kohlenstoffatomen oder
ein Mono- oder Dialkylphenylrest mit 6 bis 16 Kohlenstoffatomen je Alkylrest ist,
wobei bis zu 40 % der Reste $R^1$ durch Alkylreste mit 1 bis 4 Kohlenstoffatomen ersetzt sein können,

n einen Wert von 2, 3 oder 4 hat und im durchschnittlichen Molekül einen mittleren Wert von 2,0 bis 2,5 aufweist, und

x einen Wert von 10 bis 200 hat,

in solchen Mengen, daß 5 bis 70 % der Estergruppen umgeestert werden und in Gegenwart eines an sich bekannten Umesterungskatalysators, bei Temperaturen von 70 bis 170 °C und gegebenenfalls in Gegenwart eines Lösungsmittels.

**2.** Polyacrylsäureester nach Anspruch 1, erhältlich durch Umesterung von Polyacrylsäurealkylestern mit Polyoxyalkylenmonoolen in solchen Mengen, daß 20 bis 70 %, vorzugsweise 30 bis 70 % der Estergruppen umgeestert werden.

**3.** Polyacrylsäureester nach Anspruch 1 oder 2, dadurch gekennzeichnet, daß $R^1$ ein Alkylrest mit 14 bis 22 Kohlenstoffatomen ist.

4. Polyacrylsäureester nach Anspruch 1 oder 2, dadurch gekennzeichnet, daß $R^1$ ein Alkenylrest mit 8 bis 18 Kohlenstoffatomen ist.

5. Polyacrylsäureester nach Anspruch 1 oder 2, dadurch gekennzeichnet, daß $R^1$ ein Monoalkylphenylrest ist, dessen Alkylgruppe 6 bis 12 Kohlenstoffatome aufweist.

6. Polyacrylsäureester nach einem oder mehreren der vorhergehenden Ansprüche, dadurch gekennzeichnet, daß man die Umesterung mit Polyoxymonoolen durchführt, deren Index n einen mittleren Wert von 2,0 bis 2,3 hat.

7. Polyacrylsäureester nach einem oder mehreren der vorhergehenden Ansprüche, dadurch gekennzeichnet, daß man die Umesterung mit Polyoxymonoolen durchführt, deren Index x einen mittleren Wert von 40 bis 60 hat.

8. Verwendung der Polyacrylsäureester nach einem oder mehreren der vorhergehenden Ansprüche als Emulgatoren, Solubilisierungsmittel und als Verdickungsmittel für wäßrige, aniontensidhaltige Zubereitungen, insbesondere in der Kosmetik und Körperpflege.

## Claims

1. Polyacrylic acid esters containing long-chain alkoxylated hydrocarbonyloxy groups, obtainable by transesterification of alkyl polyacrylates obtained by free-radical polymerization, whose alkyl groups have 1 to 8 carbon atoms, it being possible for up to 50% of the acrylic acid esters to be replaced by the corresponding methacrylic acid esters, with polyoxyalkylene monools of the general average formula

$$R^1O\text{-}(C_nH_{2n}O\text{-})_xH$$

where

$R^1$ in the polymer can be identical or different and is an alkyl radical having 8 to 30 carbon atoms,
an alkenyl radical having 8 to 22 carbon atoms or a mono- or dialkylphenyl radical having 6 to 16 carbon atoms per alkyl radical,
where up to 40% of the radicals $R^1$ can be replaced by alkyl radicals having 1 to 4 carbon atoms,

n has a value of 2, 3 or 4 and in the average molecule has a mean value of 2.0 to 2.5, and

x has a value of from 10 to 200,

in amounts such that 5 to 70% of the ester groups are transesterified and in the presence of a transesterification catalyst known per se, at temperatures of from 70 to 170 °C and optionally in the presence of a solvent.

2. Polyacrylic acid esters according to Claim 1, obtainable by transesterification of alkyl polyacrylates with polyoxyalkylene monools in amounts such that 20 to 70%, preferably 30 to 70%, of the ester groups are transesterified.

3. Polyacrylic acid esters according to Claim 1 or 2, characterized in that $R^1$ is an alkyl radical having 14 to 22 carbon atoms.

4. Polyacrylic acid esters according to Claim 1 or 2, characterized in that $R^1$ is an alkenyl radical having 8 to 18 carbon atoms.

5. Polyacrylic acid esters according to Claim 1 or 2, characterized in that $R^1$ is a monoalkylphenyl radical whose alkyl group has 6 to 12 carbon atoms.

6. Polyacrylic acid esters according to one or more of the preceding claims, characterized in that the transesterification is carried out using polyoxymonools whose index n has a mean value of from 2.0 to 2.3.

7. Polyacrylic acid esters according to one or more of the preceding claims, characterized in that the transesterification is carried out using polyoxymonools whose index x has a mean value of from 40 to 60.

8. Use of the polyacrylic acid esters according to one or more of the preceding claims as emulsifiers, solubilizing agents and as thickening agents for aqueous, anionic surfactant-containing preparations, in particular in cosmetics and personal hygiene.

**Revendications**

1. Polyacrylates contenant des groupes alcoxylés d'hydrocarbures et d'oxyalkylène à chaîne longue, que l'on peut obtenir par transestérification de polyacrylates d'alkyle obtenus par polymérisation radicalaire, dont les groupes alkyles comportent de 1 à 8 atomes de carbone, jusqu'à 50 % des esters acryliques pouvant être remplacés par les esters méthacryliques correspondants, avec des polyoxyalkylène-mono-ols répondant à la formule générale moyenne :

$$R^1O\text{-}(C_nH_{2n}O\text{-})_xH$$

où

$R^1$ peuvent être identiques ou différents les uns des autres dans le polymère et signifient chacun un reste alkyle ayant de 8 à 30 atomes de carbone,
un reste alkényle ayant de 8 à 22 atomes de carbone, ou un reste mono- ou dialkylphényle ayant de 6 à 16 atomes de carbone par reste alkyle,
jusqu'à 40 % des restes $R^1$ pouvant être remplacés par des restes alkyles ayant de 1 à 4 atomes de carbone,

n vaut 2, 3 ou 4 et a une valeur moyenne de 2,0 à 2,5 dans la molécule moyenne, et

x vaut de 10 à 200,

en quantités telles, que 5 à 70 % des groupes esters sont transestérifiés, et en présence d'un catalyseur de transestérification connu en soi, à des températures de 70 à 170 °C, et éventuellement en présence d'un solvant.

2. Polyacrylates selon la revendication 1, que l'on peut obtenir par transestérification de polyacrylates d'alkyle avec des polyoxyalkylène-mono-ols en quantités telles que 20 à 70 %, de préférence 30 à 70 %, des groupes esters sont transestérifiés.

3. Polyacrylates selon la revendication 1 ou 2, caractérisés en ce que $R^1$ est un reste alkyle comportant de 14 à 22 atomes de carbone.

4. Polyacrylates selon la revendication 1 ou 2, caractérisés en ce que $R^1$ est un reste alkényle comportant de 8 à 18 atomes de carbone.

5. Polyacrylates selon la revendication 1 ou 2, caractérisés en ce que $R^1$ est un reste monoalkylphényle dont le groupe alkyle comporte de 6 à 12 atomes de carbone.

6. Polyacrylates selon une ou plusieurs des revendications précédentes, caractérisés en ce qu'on effectue la transestérification avec des polyoxymonools dont l'indice n a une valeur moyenne de 2,0 à 2,3.

7. Polyacrylates selon une ou plusieurs des revendications précédentes, caractérisés en ce qu'on effectue la transestérification avec des polyoxymonools dont l'indice x a une valeur moyenne de 40 à 60.

8. Utilisation des polyacrylates selon une ou plusieurs des revendications précédentes comme des émulsifiants, des agents de solubilisation et des agents épaississants pour des compositions aqueuses contenant des agents tensio-actifs anioniques, en particulier dans des produits cosmétiques et des produits de soins corporels.